# EUROPEAN PATENT SPECIFICATION

(11) **EP 1 455 770 B1**
(45) Date of publication and mention of the grant of the patent: **20.06.2007**
(21) Application number: 02784195.6
(22) Date of filing: 12.11.2002
(51) Int. Cl.: A61K 31/137, A61K 31/5375, A61P 25/14

(54) **USE OF NOREPINEPHRINE REUPTAKE INHIBITORS FOR THE TREATMENT OF TIC DISORDERS**
VERWENDUNG VON NOREPINEPHRINWIEDERAUFNAHMEHEMMERN ZUR BEHANDLUNG VON TICKS
UTILISATION D'INHIBITEURS DE RECAPTAGE DE NOREPINEPHRINE DANS LE TRAITEMENT DE TICS

(30) Priority: 30.11.2001 US 334494 P
(43) Date of publication of application: 15.09.2004
(73) Proprietor: ELI LILLY AND COMPANY, Indianapolis, Indiana 46285 (US)
(72) Inventor: ALLEN, Albert, John, Indianapolis, IN 46228 (US); MICHELSON, David, Carmel, IN 46032 (US)
(74) Representative: Hiscock, Ian James
(86) International application number: PCT/US2002/033628
(87) International publication number: WO 2003/047560

(56) References cited:
- EP-A- 0 534 756
- WO-A-01/01973
- WO-A-01/62236
- WO-A-02/053140
- US-B1- 6 323 242
- GEHLERT D R ET AL: "(R)-THIONISOXETINE, A POTENT AND SELECTIVE INHIBITOR OF CENTRAL ANDPERIPHERAL NOREPHINEPHRINE UPTAKE" LIFE SCIENCES, PERGAMON PRESS, OXFORD, GB, vol. 56, no. 22, 1995, pages 1915-1920, XP000602398 ISSN: 0024-3205
- RATZ A M ET AL: "SNAr Reactions of 2-Haloarylsulfoxides with Alkoxides Provide a Novel Synthesis of Thiotomoxetine" TETRAHEDRON LETTERS, ELSEVIER SCIENCE PUBLISHERS, AMSTERDAM, NL, vol. 40, no. 12, 19 March 1999 (1999-03-19), pages 2239-2242, XP004157442 ISSN: 0040-4039

## Description

The invention belongs to the fields of pharmaceutical chemistry and central nervous system medicine, and provides a use for the treatment of tic disorders.

Tics are involuntary repetitive brief movements (motor tics) or sounds (vocal tics) suffered by children and adults, manifesting themselves in about 18% of all children at some time in the course of their development (Kurlan, et al., Neurology, 57, 1383-1388 (2001)). Tic disorders may be transient, presenting for less than a year, or chronic, presenting for a year or more. Tourette's syndrome is a severe tic disorder characterized by both motor and vocal tics for at least a year. Common simple tics include eye blinking, shoulder jerking, picking movements, grunting, sniffing, and barking. Complex tics include facial grimacing, arm flapping, coprolalia (use of obscene words), palilalia (repeating one's own words), or echolalia (repeating another's words or phrases) (Bagheri, et al., American Family Physician, 2263-2273 (April 15, 1999)). Tic disorders present a significant barrier to the patients' academic achievement, with such patients five times more likely to require special education services (Kurlan at 1384).

Currently, tic disorders are most commonly treated with the traditional antipsychotics, especially haloperidol. Although many patients benefit from haloperidol treatment, many experience adverse events such as sedation, akathisia, extrapyramidal symptoms, weight gain, and infrequently tardive dyskinisia (Sallee, J. Am. Acad. Child Adolesc. Psychiatry, 39(3), 292-299 (2000)). Other treatments include the traditional antipsychotic pimozide, and α₂-agonists such as clonidine and guanfacine. The atypical antipsychotics risperidone, olanzapine, and ziprasidone are also reported to be useful for the treatment of tic disorders. Although the individual pharmacological profiles of the atypical antipsychotics are distinct, they share a relatively greater affinity for the 5-HT₂ and D₂ receptors than traditional antipsychotic agents and cause a lower incidence of extrapyramidal effects.

Additional treatment options for tic disorders that are well tolerated and present fewer side effects are needed.

The present invention provides the use of a selective norepinephrine reuptake inhibitor selected from the group consisting of atomoxetine and (R)-N-methyl-3-(2-methylthiophenoxy)-3-phenylpropylamine for the preparation of a medicament useful for the treatment or prevention of tic disorders.

The present invention further provides the use of a selective norepinephrine reuptake inhibitor selected from the group consisting of atomoxetine and (R)-N-methyl-3-(2-methylthiophenoxy)-3-phenylpropylamine for the preparation of a medicament useful for the treatment of tic disorders with comorbid Attention-deficit Hyperactivity Disorder.

Many compounds, including those discussed at length below, are selective norepinephrine reuptake inhibitors, and no doubt many more will be identified in the future. In the practice of the present invention, it is intended to include reuptake inhibitors which show 50% effective concentrations of about 1000 nM or less, in the protocol described by Wong et al., Drug Development Research, 6, 397 (1985). The norepinephrine reuptake inhibitors useful for the present invention are characterized in being selective for the inhibition of neurotransmitter reuptake relative to their ability to act as direct agonists or antagonists at other receptors. It is preferred that the compounds useful for the present invention are selective for the inhibition of norepinephrine reuptake relative to direct agonist or antagonist activity at other receptors by a factor of at least ten. Preferably, compounds useful for the present invention are selective for the inhibition of norepinephrine reuptake relative to direct agonist or antagonist activity at other receptors by a factor of at least one hundred. Norepinephrine reuptake inhibitors useful for the present invention include:

Atomoxetine (formerly known as tomoxetine), (R)-(-)-N-methyl-3-(2-methylphenoxy)-3-phenylpropylamine, is usually administered as the hydrochloride salt. Atomoxetine was first disclosed in U.S. Patent #4,314,081. The word "atomoxetine" will be used here to refer to any acid addition salt or the free base of the molecule. See, for example, Gehlert, et al., Neuroscience Letters, 157, 203-206. (1993), for a discussion of atomoxetine's activity as a norepinephrine reuptake inhibitor; and

(R) -N-methyl-3- (2-methylthiophenoxy) -3-phenylpropylamine, or a pharmaceutically acceptable salt thereof. (R)-N-methyl-3-(2-methylthiophenoxy)-3-phenylpropylamine is described in U.S. Patent 5,281,624, of Gehlert, Robertson, and Wong, and in Gehlert, et al., Life Sciences, 55(22), 1915-1920, (1995). (R)-N-methyl-3-(2-methylthiophenoxy)-3-phenylpropylamine is there taught to be an inhibitor of norepinephrine reuptake in the brain.

While all compounds selected from the group consisting of atomoxetine and (R)-N-methyl-3-(2-methylthiophenoxy)-3-phenylpropylamine are useful for the present invention, certain are preferred. The use of atomoxetine hydrochloride for the present invention is the most preferred embodiment of the present invention.

It will be understood by the skilled reader that most or all of the compounds used in the present invention are capable of forming salts, and that the salt forms of pharmaceuticals are commonly used, often because they are more readily crystallized and purified than are the free bases. In all cases, the use of the pharmaceuticals described above as salts is contemplated in the description herein, and often is preferred, and the pharmaceutically acceptable salts of all of the compounds are included in the names of them. Especially preferred pharmaceutically acceptable salts are those formed with hydrochloric acid.

The dosages of the drugs used in the present invention must, in the final analysis, be set by the physician in charge of the case using knowledge of the drugs, the properties of the drugs in combination as determined in clinical trials, and the characteristics of the patient including diseases other than that for which the physician is treating the patient. General outlines of the dosages, and some preferred dosages, can and will be provided here.

Atomoxetine: In adults and older adolescents: from about 5 mg/day to about 200 mg/day; preferably in the range from about 60 to about 150 mg/day; more preferably from about 60 to about 130 mg/day; and still more preferably from about 50 to about 120 mg/day; in children and younger adolescents: from about 0.2 to about 3.0 mg/kg/day; preferably in the range from about 0.5 to about 1.8 mg/kg/day;

(R)-N-methyl-3-(2-methylthiophenoxy)-3-phenylpropylamine: from about 0.01 mg/kg to about 20 mg/kg; preferred daily doses will be from about 0.05 mg/kg to 10 mg/kg; ideally from about 0.1 mg/kg to about 5 mg/kg;

All of the compounds concerned are orally available and are normally administered orally, and so oral administration is preferred. However, oral administration is not the only route or even the only preferred route. For example, transdermal administration may be very desirable for patients who are forgetful or petulant about taking oral medicine. (R)-N-methyl-3-(2-methylthiophenoxy)-3-phenylpropylamine may also be administered by the percutaneous, intravenous, intramuscular, intranasal or intrarectal route, in particular circumstances. The route of administration may be varied in any way, limited by the physical properties of the drugs, the convenience of the patient and the caregiver, and other relevant circumstances (Remington's Pharmaceutical Sciences, 18th Edition, Mack Publishing Co. (1990)).

The pharmaceutical compositions are prepared in a manner well known in the pharmaceutical art. The carrier or excipient may be a solid, semi-solid, or liquid material that can serve as a vehicle or medium for the active ingredient. Suitable carriers or excipients are well known in the art. The pharmaceutical composition may be adapted for oral, inhalation, parenteral, or topical use and may be administered to the patient in the form of tablets, capsules, aerosols, inhalants, suppositories, solutions, suspensions, or the like.

The compounds of the present invention may be administered orally, for example, with an inert diluent or capsules or compressed into tablets. For the purpose of oral therapeutic administration, the compounds may be incorporated with excipients and used in the form of tablets, troches, capsules, elixirs, suspensions, syrups, wafers, chewing gums and the like. These preparations should contain at least 4% of the compound of the present invention, the active ingredient, but may be varied depending upon the particular form and may conveniently be between 4% to about 70% of the weight of the unit. The amount of the compound present in compositions is such that a suitable dosage will be obtained. Preferred compositions and preparations useful for the present invention may be determined by a person skilled in the art.

The tablets, pills, capsules, troches, and the like may also contain one or more of the following adjuvants: binders such as microcrystalline cellulose, gum tragacanth or gelatin; excipients such as starch or lactose, disintegrating agents such as alginic acid, Primogel, corn starch and the like; lubricants such as magnesium stearate or Sterotex; glidants such as colloidal silicon dioxide; and sweetening agents such as sucrose or saccharin may be added or a flavoring agent such as peppermint, methyl salicylate or orange flavoring. When the dosage unit form is a capsule, it may contain, in addition to materials of the above type, a liquid carrier such as polyethylene glycol or a fatty oil. Other dosage unit forms may contain other various materials that modify the physical form of the dosage unit, for example, as coatings. Thus, tablets or pills may be coated with sugar, shellac, or other coating agents. A syrup may contain, in addition to the present compounds, sucrose as a sweetening agent and certain preservatives, dyes and colorings and flavors. Materials used in preparing these various compositions should be pharmaceutically pure and non-toxic in the amounts used.

A formulation useful for the administration of R-(-)-N-methyl 3-((2-methylphenyl)oxy)-3-phenyl-1-aminopropane hydrochloride (atomoxetine) comprises a dry mixture of R-(-)-N-methyl 3-((2-methylphenyl)oxy)-3-phenyl-1-aminopropane hydrochloride with a diluent and lubricant. A starch, such as pregelatinized corn starch, is a suitable diluent and a silicone oil, such as dimethicone, a suitable lubricant for use in hard gelatin capsules. Suitable formulations are prepared containing about 0.4 to 26% R-(-)-N-methyl 3-((2-methylphen-yl)oxy)-3-phenyl-1-aminopropane hydrochloride, about 73 to 99% starch, and about 0.2 to 1.0% silicone oil. The following tables illustrate particularly preferred formulations:

| **Ingredient (%)** | **2.5 mg** | **5 mg** | **10 mg** | **18 mg** | **20 mg** | **25 mg** | **40 mg** | **60 mg** |
|---|---|---|---|---|---|---|---|---|
| R-(-)-N-methyl 3-((2-methylphenyl)oxy)-3-phenyl-1-aminopropane hydrochloride | 1.24 | 2.48 | 4.97 | 8.94 | 9.93 | 12.42 | 19.87 | 22.12 |
| Dimethicone | 0.5 | 0.5 | 0.5 | 0.5 | 0.5 | 0.5 | 0.5 | 0.5 |
| Pregelatinized Starch | 98.26 | 97.02 | 94.53 | 90.56 | 89.57 | 87.08 | 79.63 | 77.38 |

| **Ingredient (mg/capsule)** | **2.5 mg** | **5 mg** | **10 mg** | **18 mg** | **20 mg** | **25 mg** | **40 mg** | **60 mg** |
|---|---|---|---|---|---|---|---|---|
| R-(-)-N-methyl 3-((2-methylphenyl)oxy)-3-phenyl-1-aminopropane hydrochloride | 2.86 | 5.71 | 11.43 | 20.57 | 22.85 | 28.57 | 45.71 | 68.56 |
| Dimethicone | 1.15 | 1.15 | 1.15 | 1.15 | 1.15 | 1.15 | 1.15 | 1.55 |
| Pregelatinized Starch | 225.99 | 223.14 | 217.42 | 208.28 | 206.00 | 200.28 | 183.14 | 239.89 |
| **Capsule Fill Weight (mg)** | 230 | 230 | 230 | 230 | 230 | 230 | 230 | 310 |
| Capsule Size | 3 | 3 | 3 | 3 | 3 | 3 | 3 | 2 |

For the purpose of parenteral therapeutic administration, the compounds of the present invention may be incorporated into a solution or suspension. These preparations typically contain at least 0.1% of a compound of the invention, but may be varied to be between 0.1 and about 90% of the weight thereof. The amount of (R)-N-methyl-3-(2-methylthiophenoxy)-3-phenylpropylamine present in such compositions is such that a suitable dosage will be obtained. The solutions or suspensions may also include one or more of the following adjuvants: sterile diluents such as water for injection, saline solution, fixed oils, polyethylene glycols, glycerine, propylene glycol or other synthetic solvents; antibacterial agents such as benzyl alcohol or methyl paraben; antioxidants such as ascorbic acid or sodium bisulfite; chelating agents such as ethylene diaminetetraacetic acid; buffers such as acetates, citrates or phosphates and agents for the adjustment of tonicity such as sodium chloride or dextrose. The parenteral preparation can be enclosed in ampoules, disposable syringes or multiple dose vials made of glass or plastic. Preferred compositions and preparations may be determined by one skilled in the art.

The compounds of the present invention may also be administered topically, and when done so the carrier may suitably comprise a solution, ointment, or gel base. The base, for example, may comprise one or more of the following: petrolatum, lanolin, polyethylene glycols, bees wax, mineral oil, diluents such as water and alcohol, and emulsifiers, and stabilizers. Topical formulations may contain a concentration of (R)-N-methyl-3-(2-methylthiophenoxy)-3-phenylpropylamine, or its pharmaceutical salt, from about 0.1 to about 10% w/v (weight per unit volume).

### Inhibition of norepinephrine reuptake

The ability of compounds to inhibit the reuptake of norepinephrine may be measured by the general procedure of Wong, *et al., supra.*

Male Sprague-Dawley rats weighing 150-250 gm are decapitated and brains are immediately removed. Cerebral cortices are homogenized in 9 volumes of a medium containing 0.32 M sucrose and 10 mM glucose. Crude synaptosomal preparations are isolated after differential centrifugation at 1000 x g for 10 minutes and 17,000 x g for 28 minutes. The final pellets are suspended in the same medium and kept in ice until use within the same day.

Synaptosomal uptake of ³H-norepinephrine is determined as follows. Cortical synaptosomes (equvalent to 1 mg of protein) are incubated at 37°C for 5 minutes in 1 mL Krebs-bicarbonate medium containing also 10 mM glucose, 0.1 mM iproniazide, 1 mM ascorbic acid, 0.17 mM EDTA and 50 nM ³H-norepinephrine. The reaction mixture is immediately diluted with 2 mL of ice-chilled Krebs-bicarbonate buffer and filtered under vacuum with a cell harvester (Brandel, Gaithersburg, MD). Filters are rinsed twice with approximately 5 mL of ice-chilled 0.9% saline and the uptake of ³H-norepinephrine assessed by liquid scintillation counting. Accumulation of ³H-norepinephrine at 4°C is considered to be background and is subtracted from all measurements. The concentration of the test compound required to inhibit 50% of the ³H-norepinephrine accumulation (IC₅₀ values) are determined by linear regression analysis.

The present invention provides a use of a selective norepinehprine reuptake inhibitor selected from the group consisting of atomoxetine and (R)-N-methyl-3-(2-methylthiophenoxy)-3-phenylpropylamine for the manufacture of a medicament for the treatment of tic disorders. The tic disorders contemplated by the use of the present invention are classified in the Diagnostic and Statistical Manual of Mental Disorders, 4th Version, published by the American Psychiatric Association (DSM-IV). The diagnostic criteria and DSM code numbers are supplied below for the convenience of the reader.

### Diagnostic Criteria for Tourette's Disorder (DSM 307.23)

A. Both multiple motor and one or more vocal tics have been present at some time during the illness, although not necessarily concurrently.
B. The tics occur many times a day (usually in bouts) nearly every day or intermittently throughout a period of more than 1 year, and during this period there was never a tic-free period of more than 3 consecutive months.
C. The disturbance causes marked distress or significant impairment in social, occupational, or other important areas of functioning.
D. The onset is before age 18 years.
E. The disturbance is not due to the direct physiological effects of a substance (e.g., stimulants) or a general medical condition (e.g., Huntington's disease or postviral encephalitis).

### Diagnostic Criteria for Chronic Motor or Vocal Tic Disorder (DSM 307.22)

A. Single or multiple motor or vocal tics (i.e., sudden, rapid, recurrent, nonrhythmic, stereotyped motor movements or vocalizations), but not both, have been present at some time during the illness.
B. The tics occur many times a day nearly every day or intermittently throughout a period of more than 1 year, and during this period there was never a tic-free period of more than 3 consecutive months.
C. The disturbance causes marked distress or significant impairment in social, occupational, or other important areas of functioning.
D. The onset is before age 18.
E. The disturbance is not due to the direct physiological effects of a substance (e.g., stimulants) or a general medical condition (e.g., Huntington's disease or postviral encephalitis).
F. Criteria have never been met for Tourette's Disorder.

### Diagnostic Criteria for Transient Tic Disorder (DSM 307.21)

A. Single or multiple motor and/or vocal tics (i.e., sudden, rapid, recurrent, nonrhythmic, stereotyped motor movements or vocalizations).
B. The tics occur many times a day, nearly every day for at least 4 weeks, but for no longer than 12 consecutive months.
C. The disturbance causes marked distress or significant impairment in social, occupational, or other important areas of functioning.
D. The onset is before age 18.
E. The disturbance is not due to the direct physiological effects of a substance (e.g., stimulants) or a general medical condition (e.g., Huntington's disease or postviral encephalitis).
F. Criteria have never been met for Tourette's Disorder or Chronic Motor or Vocal Tic Disorder.

### Tic Disorder Not Otherwise Specified (DSM 307.20)

This category is for disorders characterized by tics that do not meet the criteria for a specific Tic Disorder. Examples include tics lasting less than 4 weeks or tics with an onset after age 18 years.

Although patients suffering from tic disorders also commonly suffer concomitantly from Attention-deficit Hyperactivity Disorder, the courses of the two disorders are distinct and they are separate clinical entities (Biederman, et al., Arch. Gen. psychiatry, 56, 842-847 (1999)). The patient will receive benefit from the use of norepinephrine reuptake inhibitors in the amelioration of the symptoms of tic disorders regardless of whether comorbid conditions are present. Furthermore, a patient suffering from tic disorders and Attention-deficit Hyperactivity Disorder will receive benefit in the amelioration of symptoms of both conditions through the use of the present invention. A further embodiment of the present invention, therefore, is a use of a selective norepinephrine reuptake inhibitor selected from the group consisting of atomoxetine and (R)-N-methyl-3-(2-methylthiophenoxy)-3-phenylpropylamine for the manufacture of a medicament for treating tic disorders with comorbid Attention-deficit Hyperactivity Disorder.

The use of the present invention is effective in the treatment of patients who are children, adolescents or adults, and there is no significant difference in the symptoms or the details of the manner of treatment among patients of different ages. In general terms, however, for purposes of the present invention, a child is considered to be a patient below the age of puberty, an adolescent is considered to be a patient from the age of puberty up to about 18 years of age, and an adult is considered to be a patient of 18 years or older.

### EXAMPLE 1

A 12-year-old male presented with ADHD and tics. The patient had a history of ADHD, motor and vocal tics, and some anxiety related movements that were either complex tics or compulsive behaviors such as are seen in OCD. At baseline, the tics included multiple motor and multiple vocal tics, some complex or in bouts. His vocal tics were especially problematic and disruptive, causing difficulties for the boy at school, in social situations, and when the family went out in public (for example, to a restaurant). The clinical global impression-severity score (CGI-S) is a seven-point scale (1=normal, 7=very severely ill) that summarizes a clinician's overall impression about how severe a patient's illness is compared to other patients the clinician has treated. Based on the mother's report, the retrospective CGI-S score for the boy's tics at baseline was 5, markedly ill. The YGTSS is the Yale Global Tic Severity Scale and summarizes the number, severity and frequency of a patient's tics. The minimum score on the YGTSS is zero and the maximum is 50. Based on the mother's description the patient's baseline YGTSS was about 30.

After starting on atomoxetine the patient had a dramatic drop in his tics. After about 2 to 3 weeks of atomoxetine treatment, the patient's mother reported a single head tic and a few eye blinking tics in the previous week, and no vocal tics. The mother also reported that the patient's complex tics or compulsive behaviors (which had involved him playing with some doors with his feet in a somewhat stereotypical manner), had also stopped. His ADHD also improved. The CGI-S score for the boy's tics based on the interview with his mother was 2, minimally ill. Based on the same interview, the YGTSS total score for the boy was 8. The clinical global impression-improvement (CGI-I) score is a seven-point scale (1=very much improve, 7=very much worse) that summarizes a clinician's overall impression about how much a patient has improved with treatment as compared to where they were at baseline. The CGI-I score for the patient's tics was 1, very much improved.

## Claims

1. Use of a selective norepinephrine reuptake inhibitor selected from the group consisting of atomoxetine and (R)-N-methyl-3- (2-methylthiophenoxy)-3-phenylpropylamine, or a pharmaceutically acceptable salt thereof, as sole active ingredient for the manufacture of a medicament for the treatment of tic disorders.

2. Use according to claim 1, wherein said selective norepinephrine reuptake inhibitor is atomoxetine.

3. Use according to Claim 2, wherein said atomoxetine is in the form of a hydrochloride salt

4. Use according to claim 1, wherein said selective norepinephrine reuptake inhibitor is (R)-N-methyl-3- (2-methylthiophenoxy)-3-phenylpropylamine.

5. Use according to Claim 4, wherein (R)-N-methyl-3- (2-methylthiophenoxy)-3-phenylpropylamine is in the form of a hydrochloride salt

6. Use according to any one of Claims 1-5, wherein Tourette's Syndrome is treated.

7. Use according to any one of Claims 1-5, wherein Chronic Tic Disorder is treated.

8. Use according to any one of Claims 1-5, wherein Transient Tic Disorder is treated.

9. Use of a selective norepinephrine reuptake inhibitor selected from the group consisting of atomoxetine and (R)-N-methyl-3- (2-methylthiophenoxy)-3-phenylpropylamine, or a pharmaceutically acceptable salt thereof, as sole active ingredient for the manufacture of a medicament for the treatment of tic disorders with comorbid Attention-deficit Hyperactivity Disorder

10. Use according to claim 9, wherein said selective norepinephrine reuptake inhibitor is atomoxetine.

11. Use according to Claim 10, wherein said atomoxetine is in the form of a hydrochloride salt.

12. Use according to claim 9, wherein said selective norepinephrine reuptake inhibitor is (R)-N-methyl-3- (2-methylthiophenoxy)-3-phenylpropylamine.

13. Use according to Claim 12, wherein (R)-N-methyl-3- (2-methylthiophenoxy)-3-phenylpropylamine is in the form of a hydrochloride salt.

## Patentansprüche

1. Verwendung eines selektiven Norepinephrinwiederaufnahmeinhibitors, der aus der Gruppe ausgewählt ist, die besteht aus Atomoxetin und (R)-N-Methyl-3-(2-methylthiophenoxy)-3-phenylpropylamin oder einem pharmazeutisch annehmbaren Salz hiervon als einziger Wirkstoff zur Herstellung eines Arzneimittels zur Behandlung von Tickstörungen.

2. Verwendung nach Anspruch 1, worin der selektive Norepinephrinwiederaufnahmeinhibitor Atomoxetin ist.

3. Verwendung nach Anspruch 2, worin das Atomoxetin in Form eines Hydrochloridsalzes vorliegt.

4. Verwendung nach Anspruch 1, worin der selektive Norepinephrinwiederaufnahmeinhibitor (R)-N-Methyl-3-(2-methylthiophenoxy)-3-phenylpropylamin ist.

5. Verwendung nach Anspruch 4, worin (R)-N-Methyl-3-(2-methylthiophenoxy)-3-phenylpropylamin in Form eines Hydrochlroidsalzes vorliegt.

6. Verwendung nach einem der Ansprüche 1 bis 5, worin das Tourette Syndrom behandelt wird.

7. Verwendung nach einem der Ansprüche 1 bis 5, worin eine chronische Tickstörung behandelt wird.

8. Verwendung nach einem der Ansprüche 1 bis 5, worin eine vorübergehende Tickstörung behandelt wird.

9. Verwendung eines selektiven Norepinephrinwiederaufnahmeinhibitors, der aus der Gruppe ausgewählt ist, welche besteht aus Atomoxetin und (R)-N-Methyl-3-(2-methylthiophenoxy)-3-phenylpropylamin oder eines pharmazeutisch annehmbaren Salzes hiervon als einziger Wirkstoff zur Herstellung eines Arzneimittels zur Behandlung von Tickstörungen mit einer co-morbiden Aufmerksamkeitsdefizithyperaktivitätsstörung.

10. Verwendung nach Anspruch 9, worin der selektive Norepinephrinwiederaufnahmeinhibitor Atomoxetin ist.

11. Verwendung nach Anspruch 10, worin Atomoxetin in Form eines Hydrochloridsalzes vorliegt.

12. Verwendung nach Anspruch 9, worin der selektive Norepinephrinwiederaufnahmeinhibitor (R)-N-Methyl-3-(2-methylthiophenoxy)-3-phenylpropylamin ist.

13. Verwendung nach Anspruch 12, worin (R)-N-Methyl-3-(2-methylthiophenoxy)-3-phenylpropylamin in Form eines Hydrochloridssalzes vorliegt.

## Revendications

1. Utilisation d'un inhibiteur de recaptage de norépinéphrine sélectif choisi dans le groupe constitué par l'atomoxétine et la (R)-N-méthyl-3-(2-méthylthiophénoxy)-3-phénylpropylamine, ou un sel pharmaceutiquement acceptable de celles-ci, en tant qu'unique ingrédient actif pour la fabrication d'un médicament destiné au traitement de tics.

2. Utilisation selon la revendication 1, dans laquelle ledit inhibiteur de recaptage de norépinéphrine sélectif est l'atomoxétine.

3. Utilisation selon la revendication 2, dans laquelle ladite atomoxétine est sous la forme d'un sel de chlorhydrate.

4. Utilisation selon la revendication 1, dans laquelle ledit inhibiteur de recaptage de norépinéphrine sélectif est la (R)-N-méthyl-3-(2-méthylthiophénoxy)-3-phénylpropylamine.

5. Utilisation selon la revendication 4, dans laquelle ladite (R)-N-méthyl-3-(2-méthylthiophénoxy)-3-phénylpropylamine est sous la forme d'un sel de chlorhydrate.

6. Utilisation selon l'une quelconque des revendications 1 à 5, dans laquelle on traite le syndrome de la Tourette.

7. Utilisation selon l'une quelconque des revendications 1 à 5, dans laquelle on traite un tic chronique.

8. Utilisation selon l'une quelconque des revendications 1 à 5, dans laquelle on traite un tic transitoire.

9. Utilisation d'un inhibiteur de recaptage de norépinéphrine sélectif choisi dans le groupe constitué par l'atomoxétine et la (R)-N-méthyl-3-(2-méthylthiophénoxy)-3-phénylpropylamine, ou un sel pharmaceutiquement acceptable de celles -ci, en tant qu'unique ingrédient actif pour la fabrication d'un médicament destiné au traitement de tics associés à un trouble de déficit de l'attention avec hyperactivité comorbide.

10. Utilisation selon la revendication 9, dans laquelle ledit inhibiteur de recaptage de norépinéphrine sélectif est l'atomoxétine.

11. Utilisation selon la revendication 10, dans laquelle ladite atomoxétine est sous la forme d'un sel de chlorhydrate.

12. Utilisation selon la revendication 9, dans laquelle ledit inhibiteur de recaptage de norépinéphrine sélectif est la (R)-N-méthyl-3-(2-méthylthiophénoxy)-3-phénylpropylamine.

13. Utilisation selon la revendication 12, dans laquelle ladite (R)-N-méthyl-3-(2-méthylthiophénoxy)-3-phénylpropylamine est sous la forme d'un sel de chlorhydrate.
